# EUROPEAN PATENT APPLICATION

(11) **EP 4 555 963 A1**
(43) Date of publication of application: **21.05.2025**
(21) Application number: 23840001.4
(22) Date of filing: 13.07.2023
(51) Int. Cl.: A61B 34/37, A61B 1/00, A61B 17/22, A61B 17/34, A61B 90/00, A61B 17/00, A61B 34/30

(54) **MOTION COMPENSATION DEVICE**

(30) Priority: 13.07.2022 KR 20220086610
(71) Applicant: ROEN Surgical, Inc., Daejeon 34051 (KR)
(72) Inventor: YANG, Un Je, Daejeon 34051 (KR); LEE, Dong Ho, Daejeon 34051 (KR); KIM, Jae Chul, Daejeon 34051 (KR); KIM, Joon Hwan, Daejeon 34051 (KR); SONG, Kyu Seob, Daejeon 34051 (KR); KIM, Joon Yeong, Daejeon 34051 (KR); KONG, Duk Yoo, Daejeon 34051 (KR); BAEK, Hyun Woo, Daejeon 34051 (KR); JANG, Jin Hyeok, Daejeon 34051 (KR); SEO, Hyeon Se, Daejeon 34051 (KR); JEON, Ji Un, Daejeon 34051 (KR); KWON, Dong Soo, Daejeon 34051 (KR)
(74) Representative: Stolmár & Partner Patentanwälte PartG mbB
(86) International application number: PCT/KR2023/010059
(87) International publication number: WO 2024/014910

(57) **Abstract**

Proposed is a motion compensation device including an overtube configured to be inserted into a human body and reach a target object that is a surgical target, and a compensation computation part configured to compute motion compensation corresponding to motion of the target object in the human body, wherein the overtube includes a surgical tool part configured to provide treatment to the target object, and corresponding to motion of the target object, a space between the target object and the surgical tool part or a space between the target object and the overtube is maintained within a predetermined range.

## Description

### Technical Field

The present disclosure relates to a motion compensation device for computing motion compensation of a surgical tool corresponding to motion of a surgical target object in a human body.

### Background Art

In the field of medicine, robotic surgery including a master station and a slave station realized independently or together has recently gained attention.

In the surgical field where flexible endoscopes are inserted without laparotomy, robotic control of the operation of a flexible endoscope may overcome problems caused by performing surgery in a constrained environment while passing through internal tissues or organs.

In particular, a flexible endoscope connected to an external slave station or peripheral devices, such as a robot arm, may have a fixed coordinate system or position, compared to a target object, such as a diseased area or organ, in a human body. However, the target object, which is a surgical target, may make regular or irregular motion over time due to human body factors, such as respiration and the heartbeat in the body, or mechanical factors, such as wire tension. Herein, it is difficult for the doctor to perform surgery viewing a screen transmitted through a photographing means, such as a camera, provided at a fore-end of an endoscope while the target object is moving.

### Disclosure

### Technical Problem

The present disclosure may provide a technology for recognizing a surgical target that is a target object's motion in a human body, and synchronizing a surgical tool part provided at a fore-end of an overtube with the motion of the target object.

### Technical Solution

According to the present disclosure, there is provided a motion compensation device including: an overtube configured to be inserted into a human body and reach a target object that is a surgical target; and a compensation computation part configured to compute motion compensation corresponding to motion of the target object in the human body, wherein the overtube includes a surgical tool part configured to provide treatment to the target object, and corresponding to motion of the target object, a space between the target object and the surgical tool part or a space between the target object and the overtube is maintained within a predetermined range.

### Advantageous Effects

The present disclosure can compute motion of a target object including a respiration period, by using image data received at predetermined time intervals from the photographing part provided at an end of the overtube.

In addition, the present disclosure can compute motion of a target object through the motion estimation modeling part when there is no input data or when there is only minimal information including a pre-input table or a preset value.

The target object, which is the surgical target, may have 3D degrees of freedom of motion, and 3D motion of the target object can be compensated for through translational motion, bending motion, or rotational motion of the surgical tool part or the overtube of the present disclosure.

Compared to a doctor performing a treatment operation, such as fragmentation, at a specific time according to motion of a target object, the present disclosure can compensate for motion of the surgical tool part or the overtube so that the distance between the surgical tool part or the surgical means and the target object, such as a stone, is maintained, thereby increasing efficiency, such as the probability or strength of hitting the stone with a fragmentation means, such as a laser, and reducing surgery time or the frequency of mucosal contacts.

The main motion of the target object may correspond to an extension direction of the surgical tool part of the present disclosure. After motion of the surgical tool part in a translational direction is compensated for, the doctor may determine whether to compensate for the remaining motion of degrees of freedom or to perform surgery, such as fragmentation, through the monitoring part, or may perform surgery after motion compensation according to the present disclosure is completed.

The monitoring part shows a video of application of motion compensation for the target object according to the present disclosure, the doctor who performing surgery can obtain motion intuition. When it is determined that motion compensation is not smoothly performed, this can be immediately recognized and additionally corrected with the naked eye, thus allowing a more efficient response to unexpected situations during surgery.

According to the present disclosure, when motion of the target object due to respiration is compensated for and the space between the target object and the overtube or the surgical tool part is maintained, the doctor no longer needs to manually maintain a constant distance to the target object or perform surgery while forcibly stopping respiration, thereby reducing the dependence on the skill level of an individual doctor, reducing the risk of surgery depending on the patient's condition, and reducing the burden on the doctor performing surgery.

### Description of Drawings

FIG. 1 is an overall explanatory diagram of a drive part and a flexible endoscope part of a motion compensation device of the present disclosure from a surgical perspective.
FIG. 2 is an explanatory diagram of a flexible endoscope part of the present disclosure.
FIG. 3 is a configuration diagram of a motion compensation method of the present disclosure.
FIG. 4 is a configuration diagram of a motion compensation device of the present disclosure.
FIG. 5 is an explanatory diagram of motion of an overtube or a surgical tool part of the present disclosure.
FIG. 6 is an explanatory diagram of motion compensation computation of a compensation computation part of the present disclosure.
FIG. 7 shows relative motion of a target object with respect to an overtube of the present disclosure.
FIG. 8 is an explanatory diagram of orientations of the present disclosure.
FIG. 9 is an explanatory diagram of a case in which a target of motion compensation or a driving target of motion compensation is a surgical tool part according to motion of a target object of the present disclosure.
FIG. 10 is an explanatory diagram of a case in which a target of motion compensation or a driving target of motion compensation is an overtube according to motion of a target object of the present disclosure.
FIG. 11 is an explanatory diagram of the flow of received first data to fourth data of the present disclosure.
FIG. 12 is an explanatory diagram of data collection or respiration motion computation by a photographing part of the present disclosure.
FIG. 13 is an explanatory diagram of an embodiment of computation of respiration motion, such as a respiration period, by a compensation computation part of the present disclosure.
FIG. 14 shows an embodiment of obtaining third data of the present disclosure from a respirator/anesthesia workstation, which is an embodiment of a measurement part.
FIG. 15 shows another embodiment of obtaining third data of the present disclosure from a C-arm, which is another embodiment of a measurement part.

### Mode for Invention

Hereinafter, embodiments of the present disclosure will be described in detail with reference to the accompanying drawings. However, the technical idea of the present disclosure is not limited to some of the embodiments described and may be realized in various forms. One or more of the elements between the embodiments may be selectively combined and/or substituted within the scope of the technical idea of the present disclosure.

In addition, terms in the embodiments of the present disclosure may be interpreted in the sense in which they would be generally understood by those skilled in the art, unless specifically defined, and generally used terms may be interpreted considering the contextual meaning of the related technology.

In addition, the terms of the embodiments of the present disclosure are for description of the embodiments and do not limit the present disclosure, and singular forms may be interpreted as including plural forms unless the context clearly indicates otherwise.

In addition, in the elements of the embodiments of the present disclosure, terms such as first, second, and third, or A, B, and C may be used, and these terms are only for distinguishing one element from other elements and does not limit order or sequence thereof.

In addition, in the embodiments of the present disclosure, when one element is described as "connected", "linked", and "coupled" to another element, it may mean that the element is directly connected, linked, or coupled to the other, and the two elements are indirectly connected, linked, or coupled to each other by intervening elements therebetween.

In addition, in the embodiments of the present disclosure, the placement, formation, and positioning of one element on, beneath, above, or under another element may mean that the element is directly or indirectly placed, formed, or positioned on, above, or under the other. Expressions such as up or down, and an upper or lower side may mean not only an upward direction but also a downward direction with respect to one element.

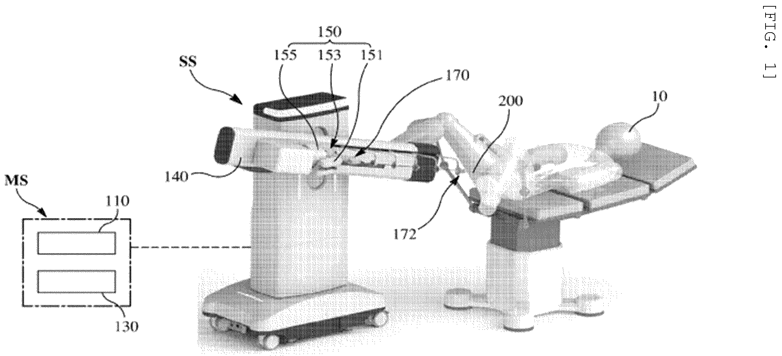

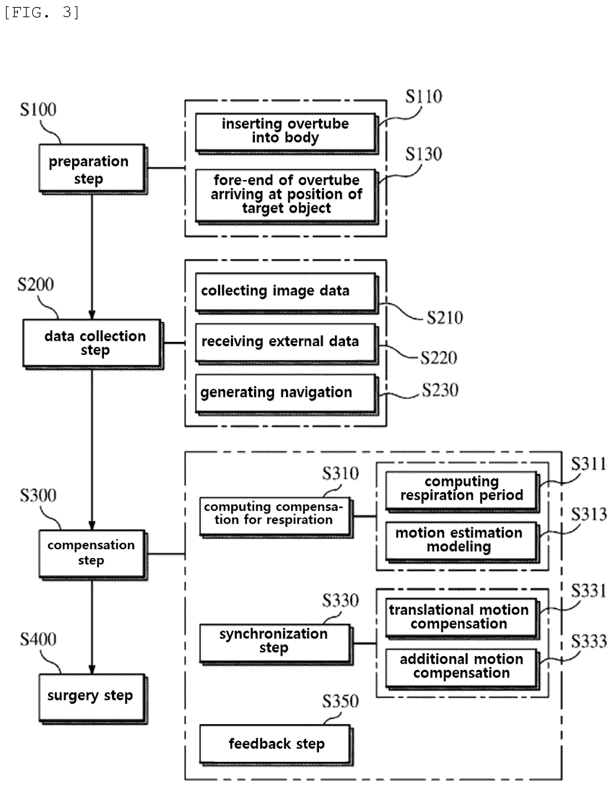

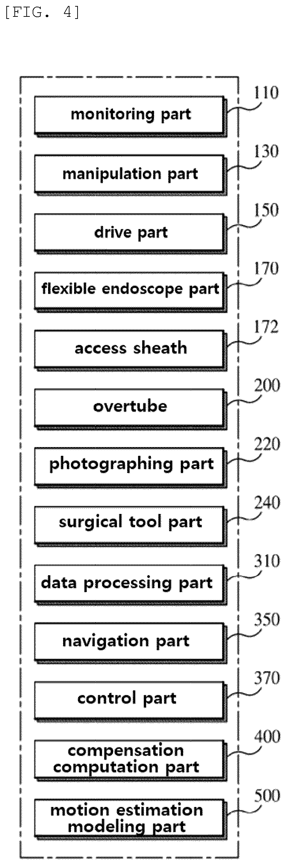

A motion compensation device and a motion compensation method of the present disclosure will be described with reference to FIGS. 1 to 15.

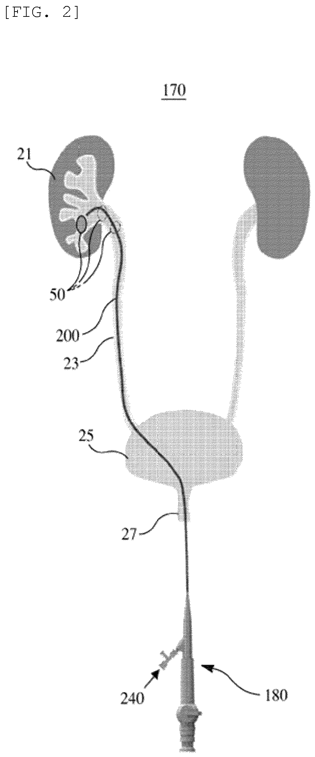

Referring to FIGS. 1 and 2, a motion compensation device 100 of the present disclosure may be applied to surgery in which a flexible endoscope part 170 is inserted into a human body.

In an embodiment, the present disclosure may be applied to retrograde intrarenal surgery (RIRS) that inserts an overtube 200 through a urethra 27 to fragment stones near a kidney 21. The present disclosure may be applied to fragmentation or extraction surgery for at least one of kidney stones, renal pelvis stones, and ureteral stones.

Herein, a target object 50 is a portion that is a target of surgery, and may be at least a diseased area of a portion of an organ or a stone that is subjected to fragmentation or extracorporeal extraction. Motion of the target object 50 may include human body factors, such as respiration and the heartbeat in the body, or mechanical factors. In an embodiment, main motion compensation of the present disclosure may mean compensation for motion of the target object 50 due to respiration, and compensation for motion of the target object 50 due to other human body factors or mechanical factors may be regarded as secondary.

The motion compensation device 100 may include a monitoring part 110 in which videos obtained through a photographing part 220 are displayed, or a manipulation part 130 that enables a doctor to operate a drive part 150 through a means including a joystick, a handle, and a gripper.

The drive part 150 may include at least one selected from the group of a translation drive part 151, a bending drive part 153, and a rotation drive part 155, and a target of the driving may be at least one selected from the group of the overtube 200, the photographing part 220, and a surgical tool part 240.

The photographing part 220 or the surgical tool part 240 may be tubular in shape and extend along the interior length of the overtube 200.

In an embodiment, the motion compensation device 100 may include a master station (MS) or a slave station (SS).

The master station (MS) may allow a doctor performing surgery to monitor the surgery situation, and may manipulate at least one selected from the group of the flexible endoscope part 170, the overtube 200, the photographing part 220, and the surgical tool part 240. The slave station SS may be operated by receiving a doctor's instructions from the master station (MS), or may be operated through a separate manipulation part that may be in conjunction with other display means.

The master station (MS) or the slave station (SS) may be realized independently or together according to the surgery site or component arrangement. The monitoring part 110 or the manipulation part 130 may be included in the master station (MS). The drive part 150, the flexible endoscope part 170, or a robot arm 140 may be included in the slave station (SS).

The flexible endoscope part 170 may be provided at the slave station (SS) including the robot arm 140, and may be adjusted by the robot arm 140 to a height or angle suitable for surgery.

The flexible endoscope part 170 may include the overtube 200 of a flexible tube type inserted into the human body in step S110. The overtube 200 may consist of a plurality of joints having degrees of freedom under predetermined constraints. As a result, an end (end tip) of the overtube 200 may freely approach the target object 50 with 3D degrees of freedom.

In an embodiment, the overtube 200 may be inserted into the urethra 27 and reach the kidney 21 or the vicinity thereof through the bladder 25 and the ureter 23. In this way, the overtube 200 is flexible to extend along irregular, tortuous, or tender organs, and may be capable of 3D operation by the drive part 150.

The photographing part 220 or the surgical tool part 240 may include a wire extending along the internal path of the overtube 200, and an end effector protruding from the fore-end of the overtube 200. The end effector may be a photographing means, such as a camera, which is capable of collecting image data of the body in the case of the photographing part 220. The image data collected by the photographing part 220 may comprehensively include data in 2D or 3D including RGB-D or stereo as well as data for obtaining body information through Lidar or IR, and sonar provided at the overtube 200.

In the case of the surgical tool part 240, the end effector may be a surgical means. The surgical means may include at least one selected from the group of a fragmentation means, collection means, and a suction means. The fragmentation means, such as a laser, is capable of fragmenting the target object 50. The collection means, such as a basket, is for collecting the target object 50 that needs to be extracted to the outside of the body. The suction means is for spraying water onto the target object 50 or suctioning the target object 50.

The surgical tool part 240 may provide treatment to the target object 50. Herein, 'treatment' may include any act that may be performed on the target object 50 during surgery, other than acquiring image data by the photographing part 220. Therefore, treatment may include the fragmentation means, the collection means, or the suction means by the above-described surgical means.

A portion of the flexible endoscope part 170 connected to the slave station (SS) or the drive part 150 may be referred to as a proximal portion, and a fore-end of the flexible endoscope part 170 connected to the photographing means or the surgical means may be referred to as a distal portion. The proximal portion or the distal portion may be used to refer the upstream direction or the downstream direction, respectively, on the path extending along the overtube 200.

The proximal portion of the flexible endoscope part 170 may be provided with a handle part 180 or an access sheath 172, and a manipulation part.

The handle part 180 may include an insertion means into which the photographing part 220 or the surgical tool part 240 may be inserted, or a manipulation means capable of manipulating at least one selected from the group of the overtube 200, the photographing part 220, and the surgical tool part 240. For example, the photographing part 220 may be provided integrated with the overtube 200, and the surgical tool part 240 may be inserted or replaced through the handle part 180.

The present disclosure may use image data obtained from the photographing part 220 to perform motion compensation including compensation for motion of the target object 50 due to respiration. The photographing part 220 or the surgical tool part 240 may be included in the overtube 200 or may be provided separately. The drawings of the present disclosure show an embodiment in which the photographing part 220 or the surgical tool part 240 is included in the overtube 200. However, no limitation thereto is imposed, and the present disclosure may be applicable to the case in which the photographing part 220 or the surgical tool part 240 is provided separately from the overtube 200.

The surgical tool part 240 having a laser and a basket may be replaced through the insertion means of the handle part 180 as the surgery progresses, or a plurality of the surgical tool parts 240 may be provided in thickness permitted by the insertion environment.

The manipulation means of the handle part 180 may be provided separately from the drive part 150 to assist the drive part 150.

The access sheath 172 may be provided at the urethra entrance, and may guide the overtube 200 to the inside of the human body. If there is no access sheath 172, the insertion or withdrawal of the overtube 200 is prone to cause damage due to organ friction. In particular, according to the present disclosure, corresponding to motion of the target object 50, the surgical means or the surgical tool part 240 may be operated to perform motion compensation.

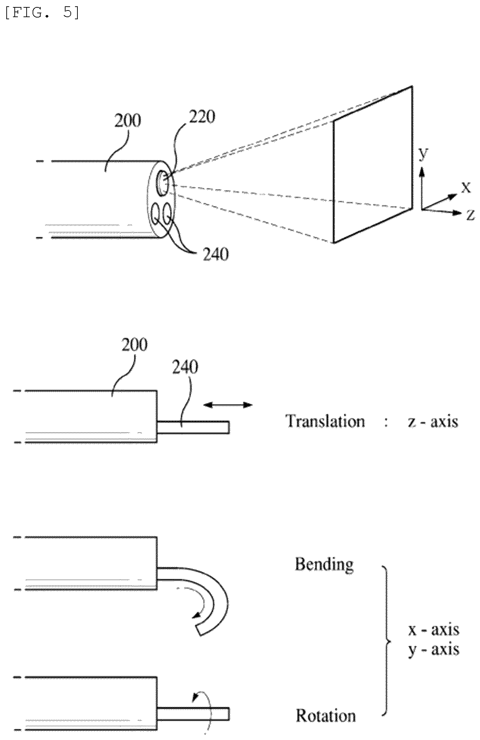

Referring to FIGS. 5 to 10, the mechanism and structure of motion compensation, such as respiration compensation and heart rate compensation, of the present disclosure will be described.

According to FIG. 5, the surgical tool part 240 of the overtube 200 may make translational motion, bending motion, or rotational motion, and through at least some thereof or combinations thereof, the surgical tool part 240 may realize any degrees-of-freedom motion in 3D. Accordingly, the fore-end of the overtube 200 may approach the target object 50 in any desired direction, passing through inside tortuous organs.

Referring to FIG. 5, it is shown that the surgical tool part 240 makes translation, bending, or rotation (rolling). However, according to the structure, an embodiment in which the translation, bending, or rotation of the overtube 200 is transmitted to the surgical tool part 240, resulting in translation, bending, or rotation (roll) of the surgical tool part 240 may be included.

An image or a video obtained by the photographing part 220 or the photographing means may be determined according to the direction the fore-end of the overtube 200 faces. Image data including a 2D video of the photographing part 220 may be realized as a coordinate system composed of an axis (z-axis) parallel to the direction that the photographing part 220 faces, and a plane (x-y plane) perpendicular to the axis. The coordinate system of a 2D image of the photographing part 220 may be referred to as a relative coordinate system that varies with the position of the fore-end of the overtube 200.

A primary objective of the present disclosure may be to maintain the change in the space between the target object 50 and the surgical tool part 240, such as a laser, and a basket, which may be provided at the fore-end of the overtube 200, that is, the distal portion, or between the overtube 200 and the target object 50 to or less than a particular level, or to maintain the space within a predetermined range.

In most cases, the objective will be to maintain the space between the surgical tool part 240 and the target object 50 within a predetermined range. However, when rather than the suction means of the surgical tool part 240, the surgical tool part 240 is detachably attached and an empty tube (or an empty channel) is used to suction at least portion of the target object 50, it may be necessary to maintain the space between the target object 50 and the overtube 200 or the fore-end of the overtube 200 within a predetermined range.

A compensation computation part 400 or a control part 370 may maintain the space between the surgical tool part 240 and the target object 50 or the space between the overtube 200 and the target object 50 within a predetermined range space. The compensation computation part 400 or the control part 370 may determine that there is normal motion compensation when the space between the surgical tool part 240 and the target object 50 or the space between the overtube 200 and the target object 50 is maintained within a predetermined set range. When it is determined that the space is out of the predetermined set range, motion compensation may be computed again.

Unless otherwise distinguished or noted herein, maintaining the space between the surgical tool part 240 and the target object 50 within a predetermined range and maintaining the space between the overtube 200 and the target object 50 within a predetermined range space are the common objectives of the present disclosure.

The distance between the surgical tool part 240 and the target object 50 is a vector concept and may have all 3D components, and motion of the target object 50 may have regularity due to its characteristic and may have repetitive motion along a specific direction. Herein, the position of the target object 50, such as a stone, attached to the body may be assumed to follow body motion, such as respiration. For example, when the position of the target object 50 is changed or makes irregular motion with each respiration, additional determination of motion may be required for compensation therefor.

The main motion direction or motion compensation direction of the target object 50 may vary with the position of the target object 50 or the position of the overtube 200.

Thus, performing motion compensation in such a manner that the direction of the fore-end of the overtube 200 matches specific direction motion of the target object 50 as much as possible may have the effect of offsetting most motion of the target object 50.

The present disclosure may be intended to provide convenience for a doctor performing surgery with a surgery robot, and may have the effect of offsetting most motion of the target object 50 such that the space between the target object 50 and the surgical tool part 240, the space between the target object 50 and the overtube 200, or the space between the target object 50 and the photographing part 220 is well maintained within a predetermined range when compensating for and offsetting the most main motion direction even if not completely offsetting motion of the target object 50. An operation for making motion compensation direction of the surgical tool part 240, the overtube 200, or the photographing part 220 match or parallel to the most main direction of motion of the target object 50 may be added.

The translational motion direction is an embodiment of the main motion direction of the target object 50, and the process for motion compensation (or main motion direction) may vary with the relative view state or position of the overtube 200 positioned in an organ, such as a kidney.

Referring to the relative coordinate system of the photographing part 220, when motion compensation according to a translational motion direction (z-axis direction) is performed on the surgical tool part 240 corresponding to motion of the target object 50 in step S331, the largest of motion of the target object 50 may be offset. The degrees of freedom according to the plane (x-y plane) perpendicular to the remaining translational motion direction may be further subjected to compensation through bending motion or rotational motion in step S333. That is, when z-axis motion, which is the translational motion direction, is subjected to compensation and offset, only motion in the plane composed of the x-axis and the y-axis may be visible.

The additional motion compensation S333 may include compensation for motion caused by mechanical factors, such as wire tension, in addition to human body factors due to factors, such as friction, restraint, and elastic deformation of tissues because of tortuous internal tissues or organs, other than the main motion direction. An additional motion compensation direction may vary with the position of the target object 50 or the position of the overtube 200.

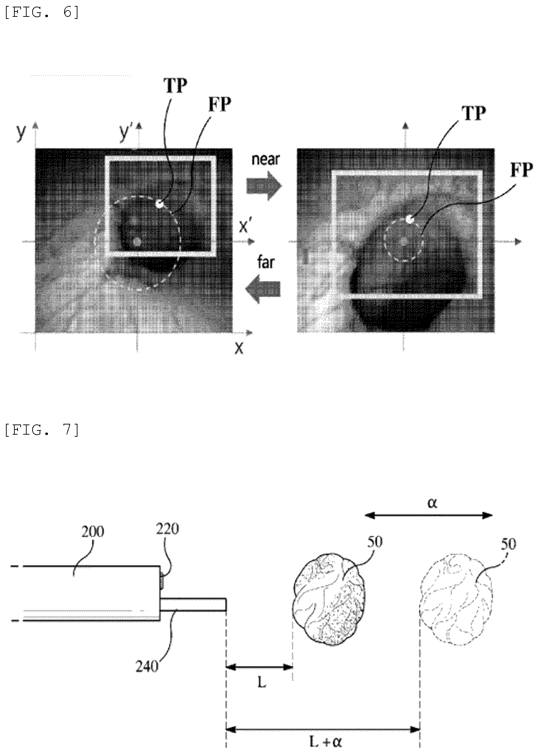

A motion compensation mechanism of the present disclosure will be described with reference to FIGS. 7 to 10.

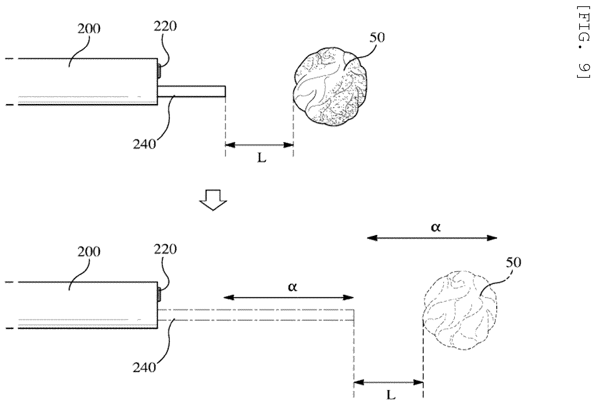

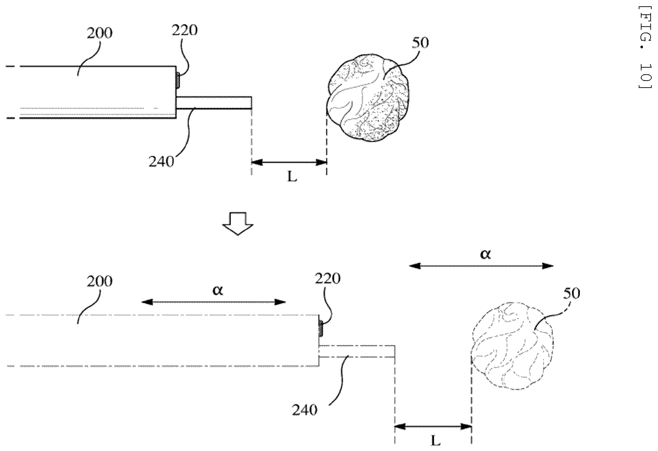

FIG. 7 shows that the fore-end of the overtube 200 reaches the target object 50, such as a targeted stone, in step S130. FIGS. 9 and 10 show that motion compensation is performed to synchronize motion between the target object 50 and the surgical tool part 240 or the overtube 200.

The overtube 200 may be inserted into the body in step S110, and may arrive at the target object 50 through translational, bending, or rotational motion in step S130. Image data may include image data, such as photographs, and videos, which a doctor monitors while performing surgery.

Herein, the photographing part 220 may collect image data at a fixed position. Herein, fixing may mean stopping with respect to an absolute coordinate system associated with extracorporeal surgery equipment, such as the master station (MS) and the slave station (SS), independent from motion of the human body.

In order to maintain the distance between the surgical tool part 240 and the target object 50 within a predetermined range by synchronizing dynamic motion of the target object 50 over time with the surgical tool part 240 for motion compensation, an absolute coordinate system that may be a reference for motion of the target object 50 is required. Unlike existing endoscopic surgery, endoscopic surgery using a robot enables reliable fixing of the position of the end effector at the end of the overtube 200, resulting in accurate motion compensation.

When the surgical tool part 240 or the fore-end of the overtube 200 reaches a target point and is fixed and stopped, as shown in FIG. 7, the distance (L+α) between the surgical tool part 240 and the target object 50 changes as the target object moves (α).

In FIG. 9 or FIG. 10, with motion compensation of the present disclosure applied, change in the distance between the surgical tool part 240 and the target object 50 may be maintained to or less than a particular value or may be maintained within a predetermined range (L-β to L+β, wherein, β is an error).

The motion direction may also vary with the position of the target object 50 even within the same organ, such as a kidney, so the view angle of the photographing part 220 may need to be changed as the target object 50 is changed. The size of motion for compensation or the degree of observed motion, and the direction of motion may be changed accordingly.

When due to organ structure, the view direction of the photographing part 220 viewed from the initial position (P1) at which the overtube 200 approaches the target object 50 and collects data significantly deviates from the motion direction (α) of the target object 50, the orientation for changing the photographing direction of the photographing part 220 may be required.

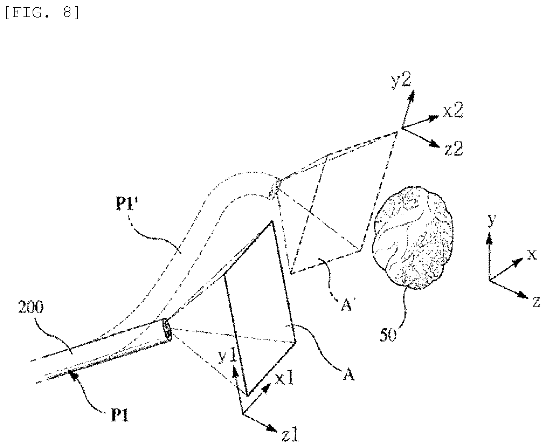

Referring to FIG. 8, when the view direction of the photographing part 220 at the initial position (P1) somewhat deviates from the motion direction (α) of the target object 50, the view direction may be matched with the motion direction (α) by being moved to a modified position (P1'). In this case, orientation adjustment may cause the relative coordinate system to be switched from the coordinate system (A) (the x1-axis, the y1-axis, and the z1-axis) of the initial position (P1) to the coordinate system (A') (the x2-axis, the y2-axis, and the z2-axis) of the modified position (P1'). The z-axis direction of the modified coordinate system (A') may be parallel to the motion direction of the target object 50. The absolute coordinate system (the x-axis, the y-axis, and the z-axis) may be incorporated in or integrated with a surgery robot or external equipment of the motion compensation device 100 which does not change with motion, such as respiration, or may be obtained through coordinate transformation into a coordinate system moving with the target object 50.

Relative coordinate values for the photographing part 220 or the target object 50 moving with respiration may be accurately computed by comparison between the absolute coordinate system and the relative coordinate system (A or A').

FIGS. 9 and 10 show an embodiment of motion compensation of the present disclosure. FIG. 9 shows that the surgical tool part 240 is synchronized corresponding to the motion (α) of the target object 50 for motion compensation. FIG. 10 shows that the overtube 200 synchronized corresponding to the motion (α) of the target object 50 for motion compensation.

When the surgical tool part 240 is synchronized, the risk of damage to organ surfaces caused by the overtube 200 may be reduced. In addition, minimizing motion on the organ surfaces may help reduce accumulation of control errors of the overtube 200 because of irregular organ internal structures.

When the overtube 200 is synchronized, a control step of issuing follow-up control instructions to the end effector of the photographing part 220 or the surgical tool part 240 in the process of performing surgery after motion compensation may be clarified.

When the surgical tool part 240 is synchronized, the surgical tool part 240 may keep making translational motion and may perform an operation for additional compensation, or follow-up instructions, such as fragmentation and collection, may be issued in an overlapping manner. However, when the overtube 200 is synchronized, the position of the photographing part 220 or the surgical tool part 240 may be fixed at the overtube 200, and when only the overtube 200 is subjected to motion compensation, the photographing part 220 or the surgical tool part 240 may automatically obtain the motion compensation effect.

The photographing part 220 may be realized in various arrangement structures, for example, the position of the photographing part is fixed at the overtube 200, or similarly to the surgical tool part 240, the photographing part operates protruding from the fore-end of the overtube 200, or the photographing part is integrated with the surgical tool part 240.

The photographing part 220 may be a target of motion compensation or a driving target of motion compensation according to motion of the target object 50.

Depending on a target of motion compensation or a driving target of motion compensation according to motion of the target object 50, and the installation position of the photographing part 220 or the arrangement structure between the overtube 200, the photographing part 220, and the surgical tool part 240, a video displayed to a doctor through the monitoring part 110 may be synchronized with the target object 50 and appear to be nearly stationary (a first video), or may cause the target object 50 to appear to be moving (a second video).

In either of the two types (the first video and the second video) of mutually stationary and mutually moving videos, the distance between the surgical tool part 240 and the target object 50 remains constant, so one of the two types may be displayed manually or automatically depending on the preference of a doctor performing surgery or the situation during surgery. An option for switching between the two types of videos according to a doctor's choice may be provided. Depending on the mutual arrangement structure between the overtube 200 and the photographing part 220 or the surgical tool part 240, switching between the first video and the second video may be achieved through video data compensation.

When motion of the surgical tool part 240 is compensated for (FIG. 9) and the position of the photographing part 220 is fixed at the overtube 200, a video displayed to a doctor through the monitoring part 110 may cause the target object 50 to appear to be moving in accordance with a respiration period and a respiration direction. In order for the target object 50 to be displayed on the monitoring part 100 as being fixed, additional image-based calculation may be required.

When motion of the surgical tool part 240 is compensated for (FIG. 9) and the photographing part 220 is not fixed at the overtube 200 and is capable of separate operation, in order for a video displayed to a doctor through the monitoring part 110 to cause the target object 50 to appear to be stationary, synchronization between the photographing part 220 and the target object 50 may be performed by additional synchronization between the photographing part 220 and the surgical tool part 240.

When motion of the surgical tool part 240 is compensated for (FIG. 9) and the photographing part 220 is realized integrated with the surgical tool part 240, a video displayed to a doctor through the monitoring part 110 may cause the target object 50 to appear to be stationary. While motion of the surgical tool part 240 is compensated for, when fragmentation or collection is performed, the target object 50 remains stationary. However, when the surgical tool part 240 is subjected to additional compensation or there is additional motion due to a doctor's manipulation, the monitored screen may be changed.

When motion of the overtube 200 is compensated for (FIG. 10) and the position of the photographing part 220 is fixed at the overtube 200, a video displayed to a doctor through the monitoring part 110 may cause the target object 50 to appear to be stationary.

When motion of the overtube 200 is compensated for (FIG. 10) and the photographing part 220 is not fixed at the overtube 200 and is capable of separate operation, in order for a video displayed to a doctor through the monitoring part 110 to cause the target object 50 to appear to be stationary, synchronization between the photographing part 220 and the overtube 200 may be additionally required.

When motion of the overtube 200 is compensated for (FIG. 10) and the photographing part 220 is realized integrated with the surgical tool part 240, a video displayed to a doctor through the monitoring part 110 may cause the target object 50 to appear to be stationary. While motion of the surgical tool part 240 is compensated for, when fragmentation or collection is performed, the target object 50 remains stationary. However, when the surgical tool part 240 is subjected to additional compensation or there is additional motion due to a doctor's manipulation, the monitored screen may be changed.

In addition, the overtube 200 or the surgical tool part 240 may be individually subjected to motion compensation, or both may be subjected to motion compensation for operation in conjunction with each other. That is, the space between the target object 50 and the surgical tool part 240 or the space between the target object 50 and the overtube 200 may be maintained within a predetermined range by interoperation of the overtube 200 and the surgical tool part 240.

The motion compensation device 100 of the present disclosure may include at least one selected from the group of a data processing part 310, a navigation part 350, the compensation computation part 400, and a motion estimation modeling part 500.

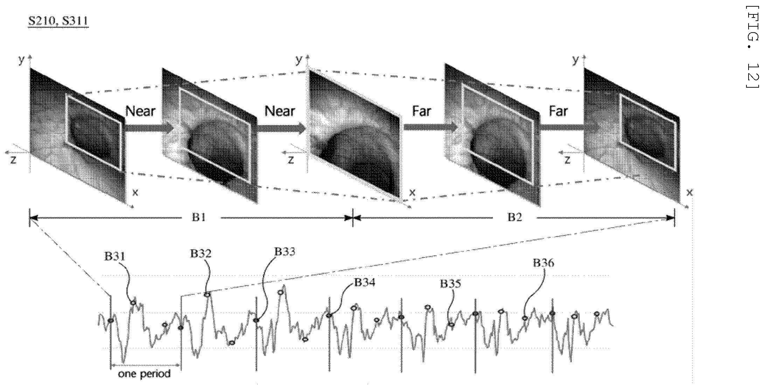

Referring to FIGS. 6 and 12, an image obtained by the photographing part 220 is expressed in the relative coordinate system of the photographing part 220. For example, according to a respiration period consisting of expiration and inspiration, the target object 50 may repeatedly approach or move away from the photographing part 220.

FIG. 12 shows respiration compensation as an embodiment of motion compensation.

Referring to FIG. 12, during inspiration (B1), the target object 50 may approach the photographing part 220, and during expiration (B2), the target object 50 may move away from the photographing part 220. An image or a video collected by the photographing part 220 in a time series may show a periodic pattern according to a respiration period. A waveform pattern for pressure or volume of respiration may include at least one selected from the group of an expiration section or expiration peak (B31, B32), an inspiration section or inspiration peak (B33, B34), and a section or peak (B35, B36) for functional residual capacity, which is the amount of exhaust not fully discharged. The compensation computation part 400 may make correspondence between features of the respiration waveform pattern from an image or video obtained by the photographing part 220, and may compute respiration compensation for the target object 50 due to respiration including a respiration period and a respiration direction (motion direction).

The compensation computation part 400 of the present disclosure may use 2D-type image data received at predetermined time intervals from the photographing part 220 provided at the end of the overtube 200 and may compute motion information including the distance between the surgical tool part 240 and the target object 50, or motion time of the target object 50 including a respiration period.

Referring to FIG. 6, the compensation computation part 400 may set a target portion (TP) or a feature portion (FP) in an image obtained by the photographing part 220. The target portion (TP) may be a reference portion, such as the target object 50, on the screen, and the feature portion (FP) may measure or express change in the position of the target portion (TP) in an image obtained over time.

The compensation computation part 400 may compute motion of the target object 50 by tracking the target portion (TP) or the feature portion (FP) from an image obtained at predetermined time intervals (for example, 100 ms).

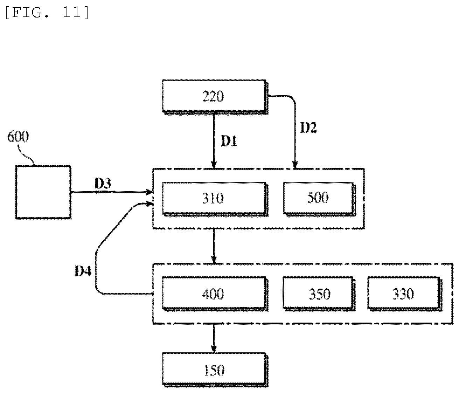

According to FIG. 11, image data obtained by the photographing part 220 may be transmitted to the data processing part 310 or the motion estimation modeling part 500.

The data processing part 310 may be provided separately from the motion estimation modeling part 500, or may be included in the motion estimation modeling part 500 and perform the same functions as if it were provided separately.

The data processing part 310 may transmit received data or data in a converted data format to the motion estimation modeling part 500.

Even when image data of the photographing part 220 or external data from a measurement part 600 of an external medical device is not input, the motion estimation modeling part 500 may use data of a motion estimation model to perform motion compensation in a specific scenario or situation. In addition, the motion estimation modeling part 500 may perform motion compensation using data pre-input in advance.

The compensation computation part 400 may receive data selectively from the data processing part 310 or the motion estimation modeling part 500, or may receive data from both.

Image data obtained by the photographing part 220 may include first data (D1) used to compensate for motion of the target object 50, such as respiration compensation, or second data (D2) continuously obtained by the photographing part 220 in real time.

The first data (D1) may include motion information of the target object 50 required for motion compensation computation of the compensation computation part 400.

The first data (D1) may include a motion period including a respiration period used to compute motion compensation of the overtube 200 or the surgical tool part 240 by the compensation computation part 400 in step S310, or information such as the target portion (TP) and the feature portion (FP).

The second data (D2) may be image data continuously obtained by the photographing part 220 in real time. The second data (D2) may conceptually overlap at least some of the first data (D1), and may be used as real-time image data collected by the photographing part 220 for monitoring a follow-up surgery process after computation of motion information including a motion period.

The second data (D2) may be used alone to compute motion compensation.

The compensation computation part 400 may receive the second data (D2) obtained in real time by the photographing part 220.

The compensation computation part 400 or the control part 370 may operate the overtube 200 or the surgical tool part 240 through the drive part 150 to compensate for motion of the target object 50 in real time by using the second data (D2) and calculating an image obtained by the photographing part 220 at predetermined time intervals through a video processing technique or a machine learning model including depth information or optical flow.

The compensation computation part 400 or the control part 370 may compute the direction in which the amount of optical flow is minimized, by using the second data (D2) observed in real time by the photographing part 220, and may adjust each joint articulation part of the overtube 200 in real time in the computed direction.

Without the need for a series of actions of computing a respiration period using the first data (D1), computing the distance between the surgical tool part 240 and the target object 50 according to the respiration period, and compensating for translational motion of the overtube 200 or the surgical tool part 240 in step S331, and further compensating for the remaining motion in step S333, when the second data (D2) is used alone to perform motion compensation in real time, the overtube 200 or the surgical tool part 240 may be controlled to compensate for motion of the target object 50 in real time.

The data processing part 310 may receive the third data (D3) that is external data received from the external measurement part 600 rather than the motion compensation device 100 including the photographing part 220.

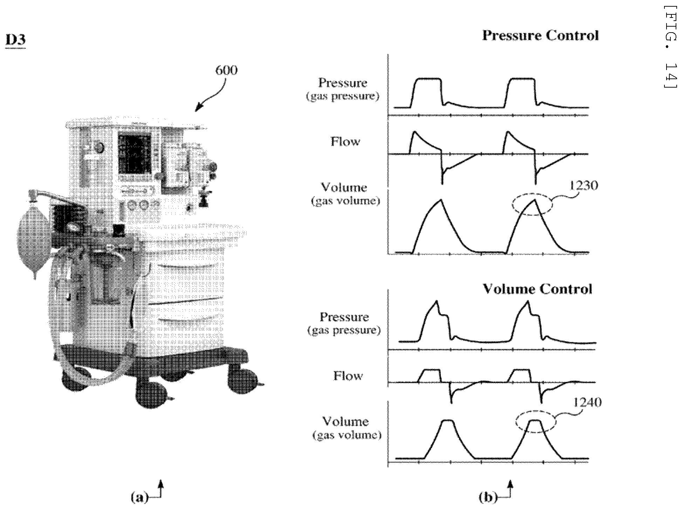

FIG. 14(a) may show an artificial respiration/anesthesia workstation, and FIG. 14(b) may include patient information, such as respiration pressure or respiration volume, which may be obtained from the artificial respiration/anesthesia workstation.

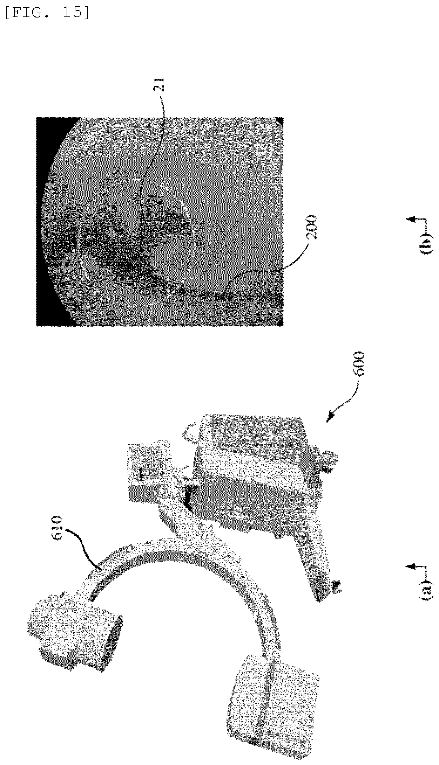

Referring to FIGS. 14 and 15, the third data (D3) may include data (D3) transmitted from the measurement part 600 of the external medical device, such as an artificial respiration/anesthesia workstation and a C-arm 610.

The third data (D3) may include data transmitted from the measurement part 600 of the external medical device rather than the photographing part 220.

The third data (D3) transmitted from the measurement part 600 of the artificial respiration/anesthesia workstation may include information on at least one selected from the group of the respiratory volume per minute, the inspiration/expiration ratio, the respiratory rate per minute, the expiration/inspiration start time, the inspiration/expiration switching time point, and the functional residual capacity section time point.

Referring to FIG. 14(b), as an embodiment of the third data(D3) obtained from the measurement part 600, pressure control per minute or volume control per minute may be shown. The third data (D3) may include information on a respiration period computed from information on a peak (1230, 1240) for pressure or volume measured by the measurement part 600. The compensation computation part 400 may use the third data (D3) including the respiration period transmitted from the measurement part 600 of the external medical device to compute compensation for motion of the target object 50 due to respiration. The compensation computation part 400 may use the third data (D3) as an initial value or a reference value for motion compensation, and may correlate the image data (D1 or D2) collected by the photographing part 220 with the third data (D3) for motion compensation computation.

The third data (D3) transmitted from the measurement part 600 of the C-arm 610 may include the forward/backward movement time of the target object 50, the respiratory rate per minute, period information, or amplitude (the amount of forward and backward movement). The third data (D3) of the C-arm may include, as shown in FIG. 15, the shape or position of the kidney 21 or a stone 50 in the kidney. The third data D3 may be used in association with map formation information of the navigation part 350.

The third data (D3) transmitted from the measurement part 600 of the C-arm may include the degree of 2D motion of an organ or the target object 50 in a plane. Using the third data (D3), relative motion may be estimated on the basis of the thickness of the overtube 200, and the amount of motion loss of the overtube 200 due to mucous membranes in contact may be computed, and the remaining time in the surgery process (for example, the remaining time in the surgery process on the basis of the amount of the remaining stone 50) may be expected.

The third data D3 may partially overlap with the first data (D1) or the second data (D2) of the photographing part 220, or may be estimated from the first data (D1) or the second data (D2).

The third data (D3) may be provided as an initial value for respiration period computation or motion estimation modelling. In this way, when the third data (D3) is used with the first data (D1) or the second data (D2), a more accurate motion compensation value may be computed.

The data processing part 310 may receive fourth data (D4) that is feedback data transmitted from at least one selected from the group of the compensation computation part 400, the navigation part 350, and an orientation part 330.

The fourth data (D4) may include feedback data on at least one selected from the group of a difference between the adjustment of the view direction of the fore-end of the overtube 200 or the photographing part 220 by the orientation part 330 and the actual view direction, a difference between motion compensation of the overtube 200 or the surgical tool part 240 by the compensation computation part 400 and the actual motion compensation, and a difference between motion compensation for each position of the target object 50 in the organ by the navigation part 350 and the actual motion compensation.

The compensation computation part 400 may compute motion compensation again by correcting a difference between the actual value and a value computed using at least one selected from the group of the first data (D1) to the fourth data (D4) or estimated from the motion estimation modeling part 500.

Even when there is no input data or there is insufficient input data, such as image data, the compensation computation part 400 may receive information from the motion estimation modeling part 500 to compute motion compensation.

The motion estimation modeling part 500 may use the fourth data (D4) to update parameters used to realize a motion estimation model.

The fourth data (D4) may include mechanical feedback information, such as information on a motor or an encoder connected to the drive part 150, information on the current state of the flexible endoscope part 170, and hysteresis or backlash information of wire tension of the photographing part 220 or the surgical tool part 240.

The data processing part 310 may switch or unify file formats so that the compensation computation part 400 uses different formats of files in an integrated manner.

The compensation computation part 400 may use data collected or stored by the data processing part 310 to compute motion compensation including respiration compensation in step S310.

The compensation computation part 400 may use at least one selected from the group of the first data (D1) to the fourth data (D4) to compensate for motion of the target object 50.

In order to compute motion compensation, such as respiration compensation, in step S310, the compensation computation part 400 may compute motion information including a respiration period in step S311.

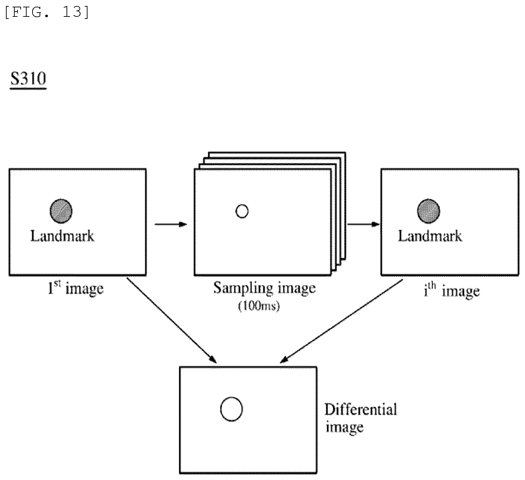

Referring to FIG. 13, when the fore-end of the overtube 200 arrives at the position of the target object in step S130, the position may be fixed and data collection by the photographing part 220 may start in step S200.

The photographing part 220 may obtain the first video (initial video) while stopped near the target object 50, and from videos obtained after the first video, image information (for example, differential image) having a high similarity to the first video may be obtained.

The obtaining of the image information (differential image) having a high similarity may correspond to finding a sampling period when the most similar video from the repeated motion of the target object 50 observed by the photographing part 220 is obtained, or may correspond to finding an index at which the histogram of the differential image is minimized.

The compensation computation part 400 may set, as a reference for respiration, the difference between the first obtained video and the time of the video having the smallest sum value of obtained differential image, and may then perform normalization on the values measured through repeated sampling within a predetermined range.

The navigation part 350 may generate a map of a specific area or one organ obtained from the photographing part 220 or the third data (D3). Even within the same organ, 3D motion obtained through the photographing part 220 may vary with position and direction in which the overtube 200 approaches, despite motion caused by respiration of the same human body, and motion compensation information requiring compensation computed accordingly may vary.

The navigation part 350 may be in conjunction with the motion estimation modeling part 500, and may compute or estimate a motion compensation value at any location or in any situation in the area of which the map is completed.

The motion estimation modeling part 500 may perform modeling to estimate body motion including the kidney 21 and the stone 50.

Motion compensation, such as respiration compensation, should not only be used at specific locations, but should be universally applicable across all situations affected by respiration, and the situations may include close observation of a diseased area during travelling. The motion estimation modeling part 500 may provide unique motion of the target object 50 using a motion estimation model. To this end, the motion estimation modeling part 500 may learn from input data, or may use a table or set value input in advance.

The motion estimation model part 500 may use, as input data, at least one selected from the group of the first data (D1) and the second data (D2) obtained from the photographing part 220, the third data (D3), which is patient information that may be obtained from an external device, such as a respirator/anesthesia workstation or a C-arm, and the fourth data (D4), which is feedback data.

The motion estimation model part 500 may compute, on the basis of the input data, at least one selected from the group of information required for motion compensation including 3D position or speed, a respiration operation value based on motion within a respiration period, current motion state information of the target object 50, the degree of performed motion compensation, control input-related motion information including hysteresis or backlash information based on image data of the photographing part 220, and information on the shape of the overtube 200 or the position of the fore-end thereof within the target object 50.

In addition, the motion estimation model may be used when it is difficult to accurately determine motion of the target object 50 from in the field of view of the photographing part 220.

When it is possible to receive the first data (D1) or the second data (D2) through the photographing part 220, in addition to the first data (D1) or the second data (D2), a value predicted by the motion estimation model may be compared thereto. When an actual value observed through the photographing part 220 and an estimation value of the motion estimation model are different from each other too much, or when a different out of a predetermined range remains for a particular time period or longer, parameters for realizing the motion estimation model may be updated.

When there is no video information input through the photographing part 220 or the information is unavailable, the motion estimation model part 500 may apply a value of the motion estimation model as a compensation value for use in motion compensation.

Herein, the unavailable information may occur when the photographing part 220 is unable to recognize motion information of the target object 50, when it is unable to fully receive feedback about a motion compensation result, or when dangerous situations, such as collisions with mucous membranes, or abnormal situations, such as input of error data and disturbances, have influence.

For example, when the field of view of the photographing part 220 is obscured by fragmentation of the stone 50 or when it is difficult to determine motion of the target object 50 or surrounding situation, the compensation computation part 400 receives data from the motion estimation model part 500 and may compensate for motion as before or according to prediction of the motion estimation model part 500 until the view is clear again.

When the compensation computation part 400 completes motion compensation computation in step S310, the control part 370 may give instructions to the drive part 150 to perform synchronization in step S330. Regarding synchronization in step S330, first, the translation drive part 151 may perform translational motion compensation in step S331, and when second motion compensation is required, compensation for degrees of freedom for the remaining motion other than translational motion or additional motion compensation due to mechanical factors may be performed in step S333.

Features, structures, and effects described in the embodiments above are included in at least one embodiment of the present disclosure, and are not necessarily limited to only one embodiment. In addition, the features, structures, and effects of each embodiment may be combined or modified with respect to other embodiments by those skilled in the art to which the embodiments belong. Accordingly, contents related to the combination and modification should be construed as being included in the scope of the present disclosure.

In addition, although the above description focuses on the embodiments, this is only an example and does not limit the present disclosure, and those skilled in the art to which the present disclosure belongs will understand that various modifications and applications not exemplified above are possible without departing from the essential characteristics of the present disclosure. For example, each element specifically shown in the embodiments may be modified and implemented. In addition, differences related to these modifications and applications should be construed as being included in the scope of the present disclosure defined in the appended claims.

### <Description of the Reference Numerals in the Drawings>

10... patient 21... kidney
23... ureter 25... bladder
27... urethra 50... target object
100... motion compensation device 110... monitoring part
130... manipulation part 140... robot arm
150... drive part 151... translation drive part
153... bending drive part 155... rotation drive part
170... flexible endoscope part 172... access sheath
180... handle part 200... overtube
220... photographing part 240... surgical tool part
310... data processing part 330... orientation part
350... navigation part 370... control part
400... compensation computation part 500... motion estimation modeling part
600... measurement part 610... C-arm
S100... preparation step S200... data collection step
S300... compensation step S400... surgery step
D1... first data D2... second data
D3... third data D4... fourth data
MS... master station SS... slave station

### <Additional Embodiments>

Embodiment 1. A motion compensation device including: an overtube configured to be inserted into a subject, the overtube including a surgical tool; and a compensation calculation part for calculating motion compensation corresponding to motion of an object in the subject, wherein the motion compensation device controls a distance between the object and the surgical tool.

Embodiment 2. The motion compensation device according to any one of the preceding embodiments, wherein the subject is a human.

Embodiment 3. The motion compensation device according to any one of the preceding embodiments, wherein the subject is an animal.

Embodiment 4. The motion compensation device according to any one of the preceding embodiments, wherein the motion compensation controls and/or adjusts a distance between the object and the surgical tool in response to motion of the object.

Embodiment 5. The motion compensation device according to any one of the preceding embodiments, wherein the motion compensation device maintains a distance between the object and the surgical tool within a predetermined distance.

Embodiment 6. The motion compensation device according to any one of the preceding embodiments, wherein the overtube is synchronized in response to motion of the object.

Embodiment 7. The motion compensation device according to any one of the preceding embodiments, wherein the object of motion compensation is the overtube.

Embodiment 8. The motion compensation device according to any one of the preceding embodiments, wherein the motion compensation device is a surgical tool unit, and the surgical tool part is a subject configured to be synchronized in response to motion of the surgical tool.

Embodiment 9. The motion compensation device according to any one of the preceding embodiments, further including a photographing part for obtaining video data of the object.

Embodiment 10. The motion compensation device according to any one of the preceding embodiments, wherein a distance between the photographing part and the object is maintained within another predetermined distance, corresponding to motion of the object.

Embodiment 11. The motion compensation device according to any one of the preceding embodiments, wherein the overtube or the surgical tool is configured to perform a plurality of motions.

Embodiment 12. The motion compensation device according to any one of the preceding embodiments, wherein the plurality of motions include translational, bending, or rotational motion.

Embodiment 13. The motion compensation device according to any one of the preceding embodiments, wherein motion compensation is performed for a translational motion direction of the overtube with respect to the object.

Embodiment 14. The motion compensation device according to any one of the preceding embodiments, wherein motion compensation for the translational motion direction corresponds to respiration compensation.

Embodiment 15. The motion compensation device according to any one of the preceding embodiments, wherein corresponding to motion of the object, motion compensation of the surgical tool is performed first in the translational motion direction, wherein corresponding to the remaining motion of the object, the surgical tool provides additional motion compensation through bending or rotational motion.

Embodiment 16. The motion compensation device according to any one of the preceding embodiments, wherein the compensation calculation part is configured to compute motion of the object using continuous 2D video data from the photographing part.

Embodiment 17. The motion compensation device according to any one of the preceding embodiments, wherein the overtube includes the photographing part for photographing the object.

Embodiment 18. The motion compensation device according to any one of the preceding embodiments, wherein the correction calculation part is configured to compute the distance or a travel interval between the surgical tool and the object.

Embodiment 19. The motion compensation device according to any one of the preceding embodiments, wherein the compensation calculation part uses 2D image data from a motion section of the object.

Embodiment 20. The motion compensation device according to any one of the preceding embodiments, wherein the compensation calculation part is configured to compute motion compensation using at least one selected from a group of first data, second data, or third data.

Embodiment 21. The motion compensation device according to any one of the preceding embodiments, wherein the second data is captured in real time through the photographing part, and the third data includes external data transmitted from an external device, and the external device is an electronic device.

Embodiment 22. The motion compensation device according to any one of the preceding embodiments, wherein the external device includes a device other than the photographing part.

Embodiment 23. The motion compensation device according to any one of the preceding embodiments, wherein the second data forms optical flow over time, and the overtube or the surgical tool part is configured to compensate for motion of the object in real time through the optical flow of the second data.

Embodiment 24. The motion compensation device according to any one of the preceding embodiments, wherein the third data is provided as an initial value for calculating a motion period of the object, and the correction calculation part is configured to compute motion compensation by linking the video data from the photographing part and the third data, which is a video measurement method.

Embodiment 25. The motion compensation device according to any one of the preceding embodiments, further including a monitoring part configured to externally display the video data obtained by the photographing part.

Embodiment 26. The motion compensation device according to any one of the preceding embodiments, wherein when a viewing direction of the photographing part deviates from a motion direction of the object, a position of the photographing unit is moved from an initial position to a corrected position.

Embodiment 27. The motion compensation device according to any one of the preceding embodiments, wherein a viewing position at the corrected position is parallel to the motion direction of the object.

Embodiment 28. The motion compensation device according to any one of the preceding embodiments, wherein when the overtube is close to the object, a position of the photographing part is fixed at a fixed position, and the photographing part is configured to collect the video data of the object from the fixed position.

Embodiment 29. The motion compensation device according to any one of the preceding embodiments, wherein the correction computation part is configured to set a target portion or a feature portion in a captured video of the photographing part, wherein the target portion is a reference portion on the screen, which indicates the object, and the feature portion represents change in a position of the target portion in a captured image, and the compensation computation part is configured to track the target portion or the feature portion from the captured image, and compute motion of the object at predetermined time intervals.

Embodiment 30. The motion compensation device according to any one of the preceding embodiments, wherein the compensation calculation part is configured to capture an initial video of the object and obtain a differential video, wherein the differential video is obtained from a video captured after the initial video, and the differential video corresponds to a sampling period when the most similar video from repeated motion of the object observed by the photographing part is obtained.

Embodiment 31. The motion compensation device according to any one of the preceding embodiments, wherein the compensation calculation part calculates a difference between the time of the video with the smallest sum of differences and the time of the initial video, wherein the difference is a reference for respiration.

Embodiment 32. The motion compensation device according to any one of the preceding embodiments, further including a motion estimation modeling part for performing modeling to estimate motion of the object, wherein the motion estimation modeling unit is configured to learn unique motion of the object.

Embodiment 33. The motion compensation device according to any one of the preceding embodiments, wherein a parameter is updated from a difference between a value estimated by the modeling estimation modeling part and an actual value observed by the photographing part, and the motion compensation device is a device configured to re-estimate motion compensation on the basis of the updated parameters.

Embodiment 34. Use of the motion compensation device according to any one of the preceding embodiments for performing a surgical operation.

Embodiment 35. A motion compensation device system including the following.
a master device having a screen and a controller;
a motion compensation device including a correction calculation part and a surgical tool configured to be inserted into a subject; and
a drive part for receiving the motion compensation device,
wherein, in response to a signal from the master device, motion compensation is configured to control a distance between an object and a surgical tool.

Embodiment 36. The motion compensation device system according to any one of the preceding embodiments, wherein the motion compensation device includes an overtube, wherein the overtube is configured to be inserted into the subject.

Embodiment 37. The motion compensation device according to any one of the preceding embodiments, wherein the subject is a human.

Embodiment 38. The motion compensation device system according to any one of the preceding embodiments, wherein the subject is an animal.

Embodiment 39. The motion compensation device system according to any one of the preceding embodiments, wherein the motion compensation controls and/or adjusts a distance between the object and the surgical tool in response to motion of the object.

Embodiment 40. The motion compensation device system according to any one of the preceding embodiments, wherein the motion compensation device maintains a distance between the object and the surgical tool within a predetermined distance.

Embodiment 41. The motion compensation device system according to any one of the preceding embodiments, wherein the overtube is synchronized in response to motion of the object.

Embodiment 42. The motion compensation device system according to any one of the preceding embodiments, wherein the object of motion compensation is the overtube.

Embodiment 43. The motion compensation device system according to any one of the preceding embodiments, wherein the motion compensation device system is a surgical tool unit, and the surgical tool part is configured to be synchronized in response to motion of the surgical tool.

Embodiment 44. The motion compensation device system according to any one of the preceding embodiments, further including a photographing part for obtaining video data of the object.

Embodiment 45. The motion compensation device system according to any one of the preceding embodiments, wherein a distance between the photographing part and the object is maintained within another predetermined distance, corresponding to motion of the object.

Embodiment 46. The motion compensation device system according to any one of the preceding embodiments, wherein the overtube or the surgical tool is configured to perform a plurality of motions.

Embodiment 47. The motion compensation device system according to any one of the preceding embodiments, wherein the plurality of motions include translational, bending, or rotational motion.

Embodiment 48. The motion compensation device system according to any one of the preceding embodiments, wherein motion compensation is performed for a translational motion direction of the overtube with respect to the object.

Embodiment 49. The motion compensation device system according to any one of the preceding embodiments, motion compensation for the translational motion direction corresponds to respiration compensation.

Embodiment 50. The motion compensation device according to any one of the preceding embodiments, wherein corresponding to motion of the object, motion compensation of the surgical tool is performed first in the translational motion direction, wherein corresponding to the remaining motion of the object, the surgical tool provides additional motion compensation through bending or rotational motion.

Embodiment 51. The motion compensation device system according to any one of the preceding embodiments, wherein the compensation calculation part and the correction calculation part are configured to compute motion of the object using continuous 2D video data from the photographing part.

Embodiment 52. The motion compensation device system according to any one of the preceding embodiments, wherein the overtube includes the photographing part for photographing the object.

Embodiment 53. The motion compensation device system according to any one of the preceding embodiments, wherein the correction calculation part is configured to compute the distance or a travel interval between the surgical tool and the object.

Embodiment 54. The motion compensation device system according to any one of the preceding embodiments, wherein the compensation calculation part uses 2D image data from a motion section of the object.

Embodiment 55. The motion compensation device system according to any one of the preceding embodiments, wherein the compensation calculation part is configured to compute motion compensation using at least one selected from a group of first data, second data, or third data.

Embodiment 56. The motion compensation device system according to any one of the preceding embodiments, wherein the second data is captured in real time through the photographing part, and the third data includes external data transmitted from an external device, and the external device is an electronic device.

Embodiment 57. The motion compensation device system according to any one of the preceding embodiments, wherein the external device includes a device other than the photographing part.

Embodiment 58. The motion compensation device system according to any one of the preceding embodiments, wherein the second data forms optical flow over time, and the overtube or the surgical tool part is configured to compensate for motion of the object in real time through the optical flow of the second data.

Embodiment 59. The motion compensation device system according to any one of the preceding embodiments, wherein the third data is provided as an initial value for calculating a motion period of the object, and the correction calculation part is configured to compute motion compensation by linking the video data from the photographing part and the third data, which is a video measurement method.

Embodiment 60. The motion compensation device system according to any one of the preceding embodiments, further including a monitoring part configured to externally display the video data obtained by the photographing part.

Embodiment 61. The motion compensation device system according to any one of the preceding embodiments, wherein when a viewing direction of the photographing part deviates from a motion direction of the object, a position of the photographing unit is moved from an initial position to a corrected position.

Embodiment 62. The motion compensation device system according to any one of the preceding embodiments, wherein a viewing position at the corrected position is parallel to the motion direction of the object.

Embodiment 63. The motion compensation device system according to any one of the preceding embodiments, wherein when the overtube is close to the object, a position of the photographing part is fixed at a fixed position, and the photographing part is configured to collect the video data of the object from the fixed position.

Embodiment 64. The motion compensation device system according to any one of the preceding embodiments, wherein the correction computation part is configured to set a target portion or a feature portion in a captured video of the photographing part, wherein the target portion is a reference portion on the screen, which indicates the object, and the feature portion represents change in a position of the target portion in a captured image, and the object is configured to track the target portion or the feature portion from the captured image and compute motion of the object at predetermined time intervals.

Embodiment 65. The motion compensation device system according to any one of the preceding embodiments, wherein the compensation calculation part is configured to capture an initial video of the object and obtain a differential video, wherein the differential video is obtained from a video captured after the initial video, and the differential video corresponds to a sampling period when the most similar video from repeated motion of the object observed by the photographing part is obtained.

Embodiment 66. The motion compensation device system according to any one of the preceding embodiments, wherein the compensation calculation part calculates a difference between the time of the video with the smallest sum of differences and the time of the initial video, wherein the difference is a reference for respiration.

Embodiment 67. The motion compensation device system according to any one of the preceding embodiments, further including a motion estimation modeling part for performing modeling to estimate motion of the object, wherein the motion estimation modeling unit is configured to learn unique motion of the object.

Embodiment 68. The motion compensation device system according to any one of the preceding embodiments, wherein a parameter is updated from a difference between a value estimated by the modeling estimation modeling part and an actual value observed by the photographing part, and the motion compensation device is a device configured to re-estimate motion compensation on the basis of the updated parameters.

Embodiment 69. Use of the motion compensation device system according to any one of the preceding embodiments for performing a surgical surgery.

Embodiment 70. An operation method of a motion compensation device, the method including: inserting an endoscope including an overtube and a surgical tool into a subject, using a compensation calculation part for calculating motion compensation corresponding to motion of an object in the subject, and adjusting the endoscope to adjust a distance between the object and the surgical tool.

Embodiment 71. The method according to embodiment 70, wherein the distance between the object and the surgical tool is within a predetermined range.

Embodiment 72. The method according to embodiment 70 or 71, further including synchronizing the overtube in response to motion of the object.

Embodiment 73. The method according to any one of the preceding embodiments, further including a photographing part for obtaining video data of the object.

Embodiment 74. The method according to any one of embodiments 70 to 73, wherein the overtube or the surgical tool is configured to perform a plurality of motions, wherein the plurality of motions include translational motion, bending motion, or rotational motion.

Embodiment 75. The method according to any one of the preceding embodiments, further including performing motion compensation for a motion direction of the overtube with respect to the object.

Embodiment 76. The method according to any one of embodiments 70 to 75, wherein the motion compensation device, the compensation calculation part, and the correction calculation part are configured to compute motion of the object using continuous 2D video data from the photographing part.

Embodiment 77. The method according to any one of the preceding embodiments, wherein motion compensation is computed using correction calculation and at least one selected from a group of first data, second data, or third data.

Embodiment 78. The method according to any one of the preceding embodiments, wherein the second data is captured in real time through the photographing part, and the third data includes external data transmitted from an external device, and the external device is an electronic device.

Embodiment 79. The method according to any one of embodiments 70 to 78, wherein the external device includes a device other than the photographing part.

Embodiment 80. The method according to any one of embodiments 70 to 79, further including computing motion compensation by linking the video data from the photographing part and the third data using the correction computation part.

Embodiment 81. The method according to any one of embodiments 70 to 80, wherein the motion compensation device further includes a monitoring unit configured to externally display the video data obtained by the photographing part.

Embodiment 82. The method according to any one of the preceding embodiments, further including moving a position of the photographing part from an initial position to a corrected position when a viewing direction of the photographic unit deviates from a motion direction of the object.

Embodiment 83. The method according to any one of the preceding embodiments, wherein a viewing position at the corrected position is parallel to the motion direction of the object.

Embodiment 84. The method according to any one of the embodiments 70 to 83, further including fixing a position of the photographing part when the overtube is close to the object, and collecting, by the photographing part, video data of the object from the fixed position.

Embodiment 85. The method according to any one of the preceding embodiments, wherein the correction calculation part is configured to set a target portion in a captured video of the photographing part as a feature portion, wherein the target portion is a reference portion on the screen, which indicates the object, and the feature portion represents change in a position of the target portion in a captured image, and the compensation computation part is configured to track the target portion or the feature portion from the captured image, and compute motion of the object at predetermined time intervals.

Embodiment 86. The method according to any one of the preceding embodiments, wherein the compensation calculation part is configured to capture an initial video of the object and obtain a differential video, wherein the differential video is obtained from a video captured after the initial video, and the differential video corresponds to a sampling period when the most similar video from repeated motion of the object observed by the photographing part is obtained.

Embodiment 87. The method according to any one of embodiments 70 to 86, further including using the compensation calculation part to calculate a difference between the time of the video with the smallest sum of differences and the time of the initial video, wherein the difference is a reference for respiration.

Embodiment 88. The method according to any one of embodiments 70 to 87, wherein the motion compensation part further includes a motion estimation modeling part for performing modeling to estimate motion of the object, and the motion estimation modeling unit is configured to learn unique motion of the object.

Embodiment 89. The method according to any one of embodiments 70 to 88, wherein a parameter is updated on the basis of a difference between a value estimated by the modeling estimation modeling part and an actual value observed by the photographing part, and motion correction is re-estimated on the basis of the updated parameter.

Embodiment 90. The method according to any one of the preceding embodiments, wherein the object is a target of the surgical tool.

Embodiment 91. The method according to any one of embodiments 70 to 90, wherein the object may include a stone.

Embodiment 92. The method according to any one of embodiments 70 to 90, wherein the object includes at least portion of a tissue.

### <ADDITIONAL EMBODIMENTS>

Embodiment 1. A motion compensation device comprising:
an overtube configured to be inserted into a subject, the overtube comprising a surgical tool; and
a compensation calculation unit for calculating motion compensation corresponding to motion of an object in the subject,
wherein the motion compensation device controls a distance between the object and the surgical tool.

Embodiment 2. The motion compensation device according to any one of the preceding embodiments, wherein the subject is human.

Embodiment 3. The motion compensation device according to any one of the preceding embodiments, wherein the subject is an animal.

Embodiment 4. The motion compensation device according to any one of the preceding embodiments, wherein the motion compensation controls and/or adjusts a distance between the object and the surgical tool in response to motion of the object.

Embodiment 5. The motion compensation device according to any one of the preceding embodiments, wherein the motion compensation device maintains a distance between the object and the surgical tool to be within a predetermined distance.

Embodiment 6. The motion compensation device according to any one of the preceding embodiments, wherein the overtube is synchronized in response to the motion of the object.

Embodiment 7. The motion compensation device according to any one of the preceding embodiments, wherein the object of the motion compensation is the overtube.

Embodiment 8. The motion compensation device according to any one of the preceding embodiments, wherein the motion compensation device is a surgical tool unit, and
wherein the surgical tool unit is synchronized in response to movement of the subject.

Embodiment 9. The motion compensation device according to any one of the preceding embodiments, further comprising a photographing unit for obtaining image data of the object.

Embodiment 10. The motion compensation device according to any one of the preceding embodiments, wherein a distance between the photographing unit and the object is maintained to be within another predetermined distance in response to motion of the object.

Embodiment 11. The motion compensation device according to any one of the preceding embodiments, wherein the overtube or surgical tool is configured to perform a plurality of motions.

Embodiment 12. The motion compensation device according to any one of the preceding embodiments, wherein the plurality of motions comprise a translational, bending, or rotational motion.

Embodiment 13. The motion compensation device according to any one of the preceding embodiments, wherein motion compensation is performed with respect to a translational movement direction of the overtube with respect to the object.

Embodiment 14. The motion compensation device according to any one of the preceding embodiments, wherein motion compensation for the translational motion direction corresponds to breathing compensation.

Embodiment 15. The motion compensation device according to any one of the preceding embodiments, wherein, in response to motion of the object, motion compensation of the surgical tool occurs first along the translational motion direction, and
wherein, in response to remaining movement of the object, the surgical tool provides additional motion compensation through bending or rotational movement.

Embodiment 16. The motion compensation device according to any one of the preceding embodiments, further comprising a compensation calculation unit,
wherein the compensation calculation unit calculates motion of the object using continuous 2D image data from the photographing unit.

Embodiment 17. The motion compensation device according to any one of the preceding embodiments, wherein the overtube includes the photographing unit for photographing the object.

Embodiment 18. The motion compensation device according to any one of the preceding embodiments, wherein the compensation calculation unit calculates a distance or movement period between the surgical tool and the object.

Embodiment 19. The motion compensation device according to any one of the preceding embodiments, wherein the compensation calculation unit uses 2D image data from the motion period of the object.

Embodiment 20. The motion compensation device according to any one of the preceding embodiments, wherein the compensation calculation unit calculates motion compensation using at least one of first data, second data, or third data.

Embodiment 21. The motion compensation device according to any one of the preceding embodiments, wherein the first data is used for calculating motion compensation that corresponds to motion of the object,
wherein the second data is captured in real time via the photographing unit, and
wherein the third data comprises external data transmitted from an external device.

Embodiment 22. The motion compensation device according to any one of the preceding embodiments, wherein the external device comprises a device other than the photographing unit.

Embodiment 23. The motion compensation device according to any one of the preceding embodiments, wherein the second data forms an optical flow over time, and
wherein the overtube or the surgical tool unit is configured to compensate for the motion of the object in real time via the optical flow of the second data.

Embodiment 24. The motion compensation device according to any one of the preceding embodiments, wherein the third data is provided as an initial value for calculating the movement period of the object, and
wherein the compensation calculation unit calculates motion compensation by linking image data of the photographing unit and the third data.

Embodiment 25. The motion compensation device according to any one of the preceding embodiments, further comprising a monitoring unit configured to externally display the image data acquired by the photographing unit.

Embodiment 26. The motion compensation device according to any one of the preceding embodiments, wherein, when a viewing direction of the photographic unit deviates from a moving direction of the object, the position of the photographing unit is moved from an initial position to a corrected position.

Embodiment 27. The motion compensation device according to any one of the preceding embodiments, wherein the viewing position at the corrected position is parallel to the moving direction of the object.

Embodiment 28. The motion compensation device according to any one of the preceding embodiments, wherein when the overtube is proximate the object, the position of the photographing unit is fixed in a fixed location, and
wherein the photographing unit collects image data of the object from the fixed location.

Embodiment 29. The motion compensation device according to any one of the preceding embodiments wherein the compensation calculation unit sets a target portion or feature portion in a captured image of the photographing unit,
wherein the target portion is a reference part on a screen that displays the object,
wherein the feature portion indicates a position change of the target portion on the captured image, and
wherein the compensation calculation unit calculates the motion of the object by tracking the target portion or the feature portion from the captured image and according to a predetermined time interval.

Embodiment 30. The motion compensation device according to any one of the preceding embodiments, wherein the compensation calculation unit captures an initial image of the object and obtains a differential image, and
wherein the differential image is obtained from images captured after the initial image, and
wherein the differential image corresponds to a sampling period in which a most similar image appears among repeated motions of the object observed by the photographing unit.

Embodiment 31. The motion compensation device according to any one of the preceding embodiments, wherein the compensation calculation unit calculates a difference between a time of an image having a smallest sum of differences and the initial image, and
wherein said difference is a criterion for respiration.

Embodiment 32. The motion compensation device according to any one of the preceding embodiments, further comprising a motion estimation modeling unit that performs modeling to estimate motion of the object, and
wherein the motion estimation modeling unit learns inherent motion of the object.

Embodiment 33. The motion compensation device according to any one of the preceding embodiments, wherein a parameter is updated from a difference between a value estimated by the modeling estimation modeling unit and an actual value observed by the photographing unit, and
wherein the motion compensation device re-estimates motion compensation based on the updated parameters.

Embodiment 34. Use of the motion compensation device according to any one of the preceding for performing a surgical operation.

Embodiment 35. A motion compensation device system comprising:
a master device having a screen and a controller;
a motion compensation device comprising a compensation calculation unit and a surgical tool configured to be inserted into a subject; and
a drive unit for receiving the motion compensation device,
wherein, in response to a signal from the master device, the motion compensation is configured to control a distance between an object and a surgical tool.

Embodiment 36. The motion compensation device system according to any one of the preceding embodiments, wherein the motion compensation device comprises an overtube, wherein the overtube is configured to be inserted into the subject.

Embodiment 37. The motion compensation device according to any one of the preceding embodiments, wherein the subject is human.

Embodiment 38. The motion compensation device system according to any one of the preceding embodiments, wherein the subject is an animal.

Embodiment 39. The motion compensation device system according to any one of the preceding embodiments, wherein the motion compensation controls and/or adjusts a distance between the object and the surgical tool in response to motion of the object.

Embodiment 40. The motion compensation device system according to any one of the preceding embodiments, wherein the motion compensation device maintains a distance between the object and the surgical tool to be within a predetermined distance.

Embodiment 41. The motion compensation device system according to any one of the preceding embodiments, wherein the overtube is synchronized in response to the motion of the object.

Embodiment 42. The motion compensation device system according to any one of the preceding embodiments, wherein the object of the motion compensation is the overtube.

Embodiment 43. The motion compensation device system according to any one of the preceding embodiments, wherein The motion compensation device system is a surgical tool unit, and
wherein the surgical tool unit is synchronized in response to movement of the subject.

Embodiment 44. The motion compensation device system according to any one of the preceding embodiments, further comprising a photographing unit for obtaining image data of the object.

Embodiment 45. The motion compensation device system according to any one of the preceding embodiments, wherein a distance between the photographing unit and the object is maintained to be within another predetermined distance in response to motion of the object.

Embodiment 46. The motion compensation device system according to any one of the preceding embodiments, wherein the overtube or surgical tool is configured to perform a plurality of motions.

Embodiment 47. The motion compensation device system according to any one of the preceding embodiments, wherein the plurality of motions comprises a translational, bending, or rotational motion.

Embodiment 48. The motion compensation device system according to any one of the preceding embodiments, wherein motion compensation is performed with respect to a translational movement direction of the overtube with respect to the object.

Embodiment 49. The motion compensation device system according to any one of the preceding embodiments, wherein motion compensation for the translational motion direction corresponds to breathing compensation.

Embodiment 50. The motion compensation device system according to any one of the preceding embodiments, wherein, in response to motion of the object, motion compensation of the surgical tool occurs first along the translational motion direction, and
wherein, in response to remaining movement of the object, the surgical tool provides additional motion compensation through bending or rotational movement.

Embodiment 51. The motion compensation device system according to any one of the preceding embodiments, further comprising a compensation calculation unit, and
wherein the compensation calculation unit calculates motion of the object using continuous 2D image data from the photographing unit.

Embodiment 52. The motion compensation device system according to any one of the preceding embodiments, wherein the overtube includes the photographing unit for photographing the object.

Embodiment 53. The motion compensation device system according to any one of the preceding embodiments, wherein the compensation calculation unit calculates a distance or movement period between the surgical tool and the object.

Embodiment 54. The motion compensation device system according to any one of the preceding embodiments, wherein the compensation calculation unit uses 2D image data from the motion period of the object.

Embodiment 55. The motion compensation device system according to any one of the preceding embodiments, wherein the compensation calculation unit calculates motion compensation using at least one of first data, second data, or third data.

Embodiment 56. The motion compensation device system according to any one of the preceding embodiments, wherein the first data is used for calculating motion compensation that corresponds to motion of the object,
wherein the second data is captured in real time via the photographing unit, and
wherein the third data comprises external data transmitted from an external device.

Embodiment 57. The motion compensation device system according to any one of the preceding embodiments, wherein the external device comprises a device other than the photographing unit.

Embodiment 58. The motion compensation device system according to any one of the preceding embodiments, wherein the second data forms an optical flow over time, and
wherein the overtube or the surgical tool unit is configured to compensate for the motion of the object in real time via the optical flow of the second data.

Embodiment 59. The motion compensation device system according to any one of the preceding embodiments, wherein the third data is provided as an initial value for calculating the movement period of the object, and
wherein the compensation calculation unit calculates motion compensation by linking image data of the photographing unit and the third data.

Embodiment 60. The motion compensation device system according to any one of the preceding embodiments, further comprising a monitoring unit configured to externally display the image data acquired by the photographing unit.

Embodiment 61. The motion compensation device system according to any one of the preceding embodiments, wherein, when a viewing direction of the photographic unit deviates from a moving direction of the object, the position of the photographing unit is moved from an initial position to a corrected position.

Embodiment 62. The motion compensation device system according to any one of the preceding embodiments, wherein the viewing position at the corrected position is parallel to the moving direction of the object.

Embodiment 63. The motion compensation device system according to any one of the preceding embodiments, wherein when the overtube is proximate the object, the position of the photographing unit is fixed in a fixed location, and
wherein the photographing unit collects image data of the object from the fixed location.

Embodiment 64. The motion compensation device system according to any one of the preceding embodiments wherein the compensation calculation unit sets a target portion or feature portion in a captured image of the photographing unit,
wherein the target portion is a reference part on a screen that displays the object,
wherein the feature portion indicates a position change of the target portion on the captured image, and
wherein calculates the motion of the object by tracking the target portion or the feature portion from the captured image and according to a predetermined time interval.

Embodiment 65. The motion compensation device system according to any one of the preceding embodiments, wherein the compensation calculation unit captures an initial image of the object and obtains a differential image, and
wherein the differential image is obtained from images captured after the initial image, and
wherein the differential image corresponds to a sampling period in which a most similar image appears among repeated motions of the object observed by the photographing unit.

Embodiment 66. The motion compensation device system according to any one of the preceding embodiments, wherein the compensation calculation unit calculates a difference between a time of an image having a smallest sum of differences and the initial image, and
wherein said difference is a criterion for respiration.

Embodiment 67. The motion compensation device system according to any one of the preceding embodiments, further comprising a motion estimation modeling unit that performs modeling to estimate motion of the object, and
wherein the motion estimation modeling unit learns inherent motion of the object.

Embodiment 68. The motion compensation device system according to any one of the preceding embodiments, wherein a parameter is updated from a difference between a value estimated by the modeling estimation modeling unit and an actual value observed by the photographing unit, and
wherein the motion compensation device re-estimates motion compensation based on the updated parameters.

Embodiment 69. Use of the motion compensation device system according to any one of the preceding for performing a surgical operation.

Embodiment 70. A method of operating a motion compensation device, the method comprising:
inserting an endoscope comprising an overtube and a surgical tool into a subject,
utilizing a compensation calculation unit for calculating motion compensation corresponding to motion of an object in the subject, and
adjusting the endoscope to adjust a distance between the object and the surgical tool.

Embodiment 71. The method according to embodiment 70, wherein the wherein the distance between the object and the surgical tool is within a predetermined range.

Embodiment 72. The method according to embodiment 70 or 71, further comprising synchronizing the overtube in response to the motion of the object.

Embodiment 73. The method according to any one of embodiments 70-72, further comprising a photographing unit for obtaining image data of the object.

Embodiment 74. The method according to any one of embodiments 70-73, wherein the overtube or surgical tool is configured to perform a plurality of motions, and
wherein plurality of motions comprise a translational, bending, or rotational motion.

Embodiment 75. The method according to any one of embodiments 70-74, further comprising performing motion compensation with respect to a translational movement direction of the overtube with respect to the object.

Embodiment 76. The method according to any one of embodiments 70-75, wherein the motion compensation device comprises a compensation calculation unit, and
wherein the compensation calculation unit calculates motion of the object using continuous 2D image data from the photographing unit.

Embodiment 77. The method according to any one of embodiments 70-76, further using the compensation calculation to calculate motion compensation using at least one of first data, second data, or third data.

Embodiment 78. The method according to any one of embodiments 70-77, wherein the first data is used for calculating motion compensation that corresponds to motion of the object,
wherein the second data is captured in real time via the photographing unit, and
wherein the third data comprises external data transmitted from an external device.

Embodiment 79. The method according to any one of embodiments 70-78, wherein the external device comprises a device other than the photographing unit.

Embodiment 80. The method according to any one of embodiments 70-79, further comprising using the compensation calculation unit to calculate motion compensation by linking image data of the photographing unit and the third data.

Embodiment 81. The method according to any one of embodiments 70-80, wherein the motion compensation device further comprises a monitoring unit configured to externally display the image data acquired by the photographing unit.

Embodiment 82. The method according to any one of embodiments 70-81, further comprising moving the position of the photographing unit from an initial position to a corrected position when a viewing direction of the photographic unit deviates from a moving direction of the object.

Embodiment 83. The method according to any one of embodiments 70-82, wherein the viewing position at the corrected position is parallel to the moving direction of the object.

Embodiment 84. The method according to any one of embodiments 70-83, further comprising fixing the location of the photographing unit when the overtube is proximate the object, and
wherein the photographing unit collects image data of the object from the fixed location.

Embodiment 85. The method according to any one of embodiments 70-84, setting a target portion a feature portion in a captured image of the photographing unit via the compensation calculation unit,
wherein the target portion is a reference part on a screen that displays the object,
wherein the feature portion indicates a position change of the target portion on the captured image, and
wherein the compensation calculation unit calculates the motion of the object by tracking the target portion or the feature portion from the captured image and according to a predetermined time interval.

Embodiment 86. The method according to any one of embodiments 70-85, wherein the compensation calculation unit captures an initial image of the object and obtains a differential image, and
wherein the differential image is obtained from images captured after the initial image, and
wherein the differential image corresponds to a sampling period in which a most similar image appears among repeated motions of the object observed by the photographing unit.

Embodiment 87. The method according to any one of embodiments 70-86, further comprising using the compensation calculation unit to calculates a difference between a time of an image having a smallest sum of differences and the initial image, and
wherein said difference is a criterion for respiration.

Embodiment 88. The method according to any one of embodiments 70-87,
wherein the motion compensation unit further comprising a motion estimation modeling unit that performs modeling to estimate motion of the object, and
wherein the motion estimation modeling unit learns inherent motion of the object.

Embodiment 89. The method according to any one of embodiments 70-88, further comprising
updating a parameter based on a difference between a value estimated by the modeling estimation modeling unit and an actual value observed by the photographing unit, and
re-estimating motion compensation based on the updated parameters.

Embodiment 90. The method according to any one of embodiments 70-89, wherein the object is a target of the surgical tool.

Embodiment 91. The method according to any one of embodiments 70-90, wherein the object comprises a stone.

Embodiment 92. The method according to any one of embodiments 70-90, wherein the object comprises at least a part of a tissue.

## Claims

1. A motion compensation device, comprising:
an overtube configured to be inserted into a human body and reach a target object that is a surgical target; and
a compensation computation part configured to compute motion compensation corresponding to motion of the target object in the human body,
wherein the overtube comprises a surgical tool part configured to provide treatment to the target object, and
corresponding to motion of the target object, a space between the target object and the surgical tool part or a space between the target object and the overtube is maintained within a predetermined range.

2. The motion compensation device of claim 1, wherein a target of motion compensation is the overtube, and
the overtube is configured to be synchronized corresponding to motion of the target object.

3. The motion compensation device of claim 1, wherein a target of motion compensation is the surgical tool part, and
the surgical tool part is configured to be synchronized corresponding to motion of the target object.

4. The motion compensation device of claim 1, wherein targets of motion compensation are the overtube and the surgical tool part,
the overtube and the surgical tool part are configured to be interoperated corresponding to motion of the target object, and
by interoperation of the overtube and the surgical tool part, the space between the target object and the surgical tool part or the space between the target object and the overtube is maintained within the predetermined range.

5. The motion compensation device of claim 1, wherein the overtube comprises a photographing part configured to obtain image data of the target object, and
corresponding to motion of the target object, a space between the photographing part and the target object is maintained within the predetermined range.

6. The motion compensation device of claim 1, wherein the overtube or the surgical tool part are configured to make translational, bending, or rotational motion,
motion compensation is performed for a translational motion direction of the overtube or the surgical tool part with respect to the target object, and
motion compensation for the translational motion direction corresponds to motion of the target object due to respiration according to human body respiration.

7. The motion compensation device of claim 1, wherein the surgical tool part or the overtube is configured to, corresponding to motion of the target object, be first subjected to motion compensation according to a translational motion direction with respect to the target object, and
the remaining motion of the target object is subjected to additional compensation through bending or rotation of the surgical tool part or the overtube.

8. The motion compensation device of claim 1, wherein the overtube comprises a photographing part configured to photograph the target object, and
the compensation computation part is configured to compute motion of the target object using successive image data of the photographing part.

9. The motion compensation device of claim 1, wherein the overtube comprises a photographing part configured to photograph the target object,
the target object repeatedly approaches or moves away from the photographing part according to a motion period including a respiration period, and
the compensation computation part is configured to compute the space between the surgical tool part and the target object, the motion period, or a motion direction, using image data according to the motion period of the target object.

10. The motion compensation device of claim 1, wherein the overtube comprises a photographing part configured to photograph the target object,
a motion estimation modeling part configured to perform modeling to estimate motion of the target object including body motion is comprised, and
the compensation computation part is configured to receive data from the motion estimation modeling part and compute motion compensation even when there is no image data input through the photographing part or there is no data transmitted from a measurement part of an external device.

11. The motion compensation device of claim 1, wherein the overtube comprises a photographing part configured to photograph the target object,
first data is data used to compute motion compensation corresponding to motion of the target object,
second data is data obtained by the photographing part in real time,
third data is external data transmitted from a measurement part of an external device rather than the photographing part, and
the compensation computation part is configured to compute motion compensation using at least one selected from a group of the first data, the second data, and the third data.

12. The motion compensation device of claim 1, wherein the overtube comprises a photographing part configured to photograph the target object,
the compensation computation part is configured to receive second data obtained by the photographing part in real time, and
the overtube or the surgical tool part is configured to operate to compensate for motion of the target object in real time, by motion compensation of the compensation computation part using the second data.

13. The motion compensation device of claim 1, wherein the overtube comprises a photographing part configured to photograph the target object,
the compensation computation part is configured to receive third data related to patient information transmitted from a measurement part of an external device rather than the photographing part,
the third data is provided to compute a motion period of the target object including a respiration period, or to compute a motion direction, and
the compensation computation part is configured to compute motion compensation by linking image data of the photographing part and the third data.

14. The motion compensation device of claim 1, wherein the overtube comprises a photographing part configured to obtain image data of the target object,
a monitoring part configured to externally display image data obtained by the photographing part is comprised,
whether the target object is stationary or moving on the monitoring part
is determined according to whether a target of motion compensation for motion of the target object is at least one selected from a group of the overtube, the surgical tool part, and the photographing part, or
is determined according to an arrangement structure between the overtube, the photographing part, and the surgical tool part.

15. The motion compensation device of claim 1, wherein the overtube comprises a photographing part configured to photograph the target object,
the overtube or the photographing part is configured to move to a modified position when a view direction of an initial position of the photographing part deviates from a motion direction of the target object, and
the view direction of the photographing part at the modified position is set to match or be parallel to the motion direction of the target object.

16. The motion compensation device of claim 1, wherein when the overtube arrives at the target object, a position of a photographing part is fixed,
the photographing part is configured to collect, at the fixed position, image data of the target object, and
the overtube or the surgical tool part is configured to, on the basis of the image data of the photographing part, operate in synchronization with motion of the target object.

17. The motion compensation device of claim 1, wherein the overtube comprises a photographing part configured to photograph the target object,
the compensation computation part is configured to set a target portion or a feature portion in an image obtained by the photographing part,
the target portion is a reference portion on a screen including the target object,
the feature portion indicates change in a position of the target portion in the obtained image, and
the compensation computation part is configured to compute motion of the target object by tracking the target portion or the feature portion from the image obtained by the photographing part at predetermined time intervals.

18. The motion compensation device of claim 1, wherein the overtube comprises a photographing part configured to photograph the target object,
the compensation computation part is configured to obtain an initial video of the target object, and obtain image information having a high similarity to the initial video from a video obtained after the initial video, and
the compensation computation part is configured to set, as a reference of a respiration period, a difference between the initial video and time of the video having the smallest sum value of the obtained image information having a high similarity.

19. The motion compensation device of claim 1, wherein the overtube comprises a photographing part configured to photograph the target object,
a motion estimation modeling part configured to perform modeling to estimate motion of the target object including body motion is comprised,
the motion estimation modeling part is configured to learn unique motion of the target object,
the motion estimation modeling part is configured to
update a parameter from a difference between a value estimated by modeling and an actual value observed by the photographing part, and
estimate motion compensation again on the basis of the updated parameter.
